# EUROPEAN PATENT APPLICATION

(11) **EP 4 671 245 A1**
(43) Date of publication of application: **31.12.2025**
(21) Application number: 24759626.5
(22) Date of filing: 19.02.2024
(51) Int. Cl.: C07D 487/04, A61K 31/519, A61P 35/00

(54) **HETEROAROMATIC RING COMPOUND SERVING AS CDK7 KINASE INHIBITOR, PREPARATION THEREOF, AND USE THEREOF**

(30) Priority: 20.02.2023 CN 202310136881
(71) Applicant: Jiangsu Chia Tai Fenghai Pharmaceutical Co., Ltd., Yancheng, Jiangsu 224100 (CN)
(72) Inventor: ZHU, Yongqiang, Yancheng, Jiangsu 224100 (CN); JIANG, Chunhuan, Yancheng, Jiangsu 224100 (CN); TAN, Qing, Yancheng, Jiangsu 224100 (CN); SHI, Jingmiao, Yancheng, Jiangsu 224100 (CN); WANG, Yuke, Yancheng, Jiangsu 224100 (CN)
(74) Representative: Engelhard, Markus
(86) International application number: PCT/CN2024/077490
(87) International publication number: WO 2024/174949

(57) **Abstract**

The present invention provides a heteroaromatic ring compound serving as a CDK7 kinase inhibitor, preparation thereof, and a use thereof. The heteroaromatic ring compound is a compound having the structure of formula (I) or a pharmaceutically acceptable salt, ester, stereoisomer, solvent compound or prodrug thereof. The present invention also provides a pharmaceutical composition comprising the compound having the structure of formula (I) or the pharmaceutically acceptable salt, ester, stereoisomer, solvent compound or prodrug thereof. The present invention further provides a use of the compound having the structure of formula (I) or the pharmaceutical composition in the preparation of a CDK7 kinase inhibitor and in the preparation of a drug for treating and/or inhibiting CDK-related diseases. The CDK7 kinase inhibitor compound of the present invention has an enzyme and cell level biological activity superior to that of disclosed similar target compounds and lower cardiotoxicity. The compound of the present invention provides more options for novel antitumor drugs, and has good prospects of application in drugs.

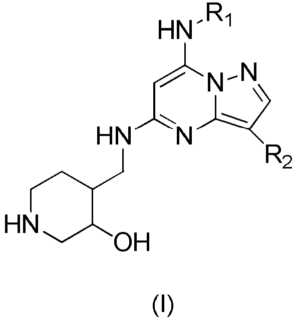

## Description

### TECHNICAL FIELD

The present invention relates to a series of derivative compounds with heteroaryl structures and their applications as CDK7 kinase inhibitors. Specifically, it relates to compounds represented by formula (I) or pharmaceutically acceptable salts thereof.

### BACKGROUND ART

Cyclin-dependent kinases (CDKs) belong to the serine/threonine kinase family. The monomers themselves are inactive and must bind to the corresponding cyclins to form active heterodimeric complexes that exert regulatory functions. These complexes can catalyze the phosphorylation of corresponding substrates, thereby directly or indirectly regulating the completion of the cell cycle and inducing cell growth and proliferation. To date, the human genome has been found to encode 21 types of CDKs and more than 15 types of cyclins. According to their functions, CDKs can be divided into two major categories: CDKs that control the cell cycle and CDKs that regulate transcription. Among them, CDK1/2/4/6 are mainly associated with the cell cycle, while CDK7/8/9/10 are primarily involved in the transcriptional mechanisms of genetic information within cells.

CDK7 is an important member of the CDKs family and primarily regulates the cell cycle through two indirect mechanisms: CDK7, together with cyclin H and Mat1, forms the CAK (CDKs activating kinase) complex, which further phosphorylates CDK1/2, thereby activating their functions in the cell cycle.

Another mechanism is that CDK7 acts as a subunit component of the general transcription factor TFIIH and phosphorylates the C-terminal domain (CTD) of the large subunit of RNA polymerase II (RNAP II), thereby regulating the gene transcription process in cells. Due to its dual function in CAK and CTD phosphorylation, CDK7 plays a vital role in cell proliferation, the cell cycle, and transcription processes.

In recent years, inhibition of CDK7 has gradually become a promising therapeutic strategy for various cancers. Inhibiting CDK7 can suppress the expression of key oncogenes such as c-Myc. Preclinical research data indicate that small molecule inhibitors of CDK7 have demonstrated good anticancer effects in hormone receptor-positive and triple-negative breast cancers, as well as transcription factor-driven cancers such as small cell lung cancer (SCLC), for example, the small molecule drug CT-7001 developed by Emory University. These cancers currently lack effective treatment options and present significant unmet medical needs. Moreover, due to their different mechanism of action, CDK7 inhibitors may also be effective against cancers that have developed resistance to current therapies. Therefore, the development of CDK7 inhibitors is likely to become an effective approach for treating these malignant tumors.

### SUMMARY

The objective of the present invention is to provide a class of heteroaryl compounds as CDK7 kinase inhibitors.

The objective of the present invention can be achieved through the following measures:
A first objective of the present invention is to provide compounds of formula (I), or pharmaceutically acceptable salts, esters, stereoisomers, solvates, or prodrugs thereof, 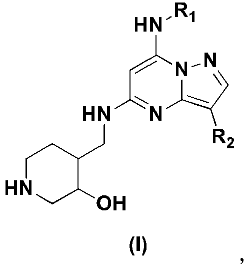 wherein, R₁ is a substituted or unsubstituted deuterated C1-C3 alkyl, C1-C3 alkyl, C2-C6 alkenyl, C2-C6 alkynyl, or a substituted or unsubstituted C6-C10 aryl-C1-C6 alkyl;

The substituent is independently selected from H, D, halogen, hydroxyl, -CN, carbonyl, C1-C3 alkyl, C2-C3 alkenyl,C2-C3 alkynyl, deuterated C1-C3 alkyl, C1-C3 alkoxy, hydroxyl-C1-C3 alkyl, fluoro-C1-C3 alkyl, cyano-C1-C3 alkyl, C3-C8 cycloalkyl, C3-C10 heterocyclyl, or C6-C10 aryl;
R₂ is selected from halogen, hydroxyl, -CN, carbonyl, C1-C3 alkyl, C1-C3 alkenyl, C1-C3 alkynyl, deuterated C1-C3 alkyl, C1-C3 alkoxy, hydroxyl-C1-C3 alkyl, fluoro-C1-C3 alkyl, cyano-C1-C3 alkyl;
When R₁ is benzyl, R₂ is -CN.

In a further embodiment, R1 is a substituted or unsubstituted C1-C3 alkyl, C2-C6 alkenyl, C2-C6 alkynyl, or a substituted or unsubstituted C6-C10 aryl-C1-C6 alkyl.

In a further embodiment, R₂ is selected from -CN and C1-C3 alkyl.

In a further embodiment, R₁ is substituted or unsubstituted

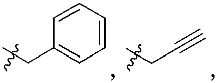

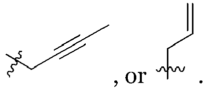

In a further embodiment, the compound is selected from:

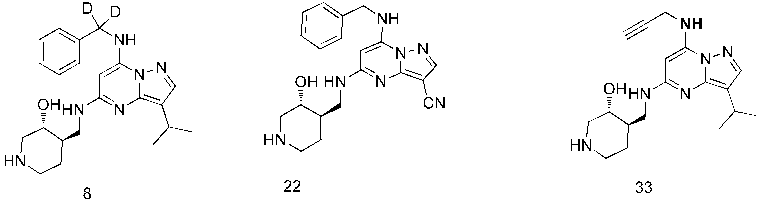

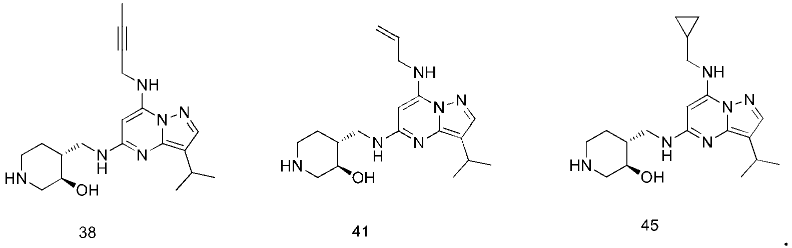

The salts that may be formed by the compounds of the present invention are also within the scope of the present invention. Unless otherwise specified, the compounds of the present invention are understood to include their salts. For example, a compound of formula (I) may react with an amount (e.g., an equivalent) of acid or base to form a salt which precipitates from the medium or is obtained by freeze-drying from an aqueous solution. The basic fragments contained in the compounds of the present invention, including but not limited to amines, pyridines, or imidazole rings, may form salts with organic or inorganic acids. Non-limiting examples of pharmaceutically acceptable salts of the compound of formula (I) include monohydrochloride, dihydrochloride, methanesulfonate, trifluoroacetate, and bis-trifluoroacetate.

The compounds of the present invention, when obtained sequentially through preparation, separation, and purification, have a weight content equal to or greater than 90%, for example, equal to or greater than 95%, or equal to or greater than 99% ("highly pure" compounds), as described in the specification. These "highly pure" compounds of the present invention are also considered part of the present invention.

A third objective of the present invention is to provide a pharmaceutical composition comprising a compound of formula (I), or a pharmaceutically acceptable salt, ester, stereoisomer, solvate, or prodrug thereof.

Further, the pharmaceutical composition comprises other therapeutic agents and/or pharmaceutically acceptable carriers.

A fourth objective of the present invention is to provide the use of a compound of formula (I), or a pharmaceutically acceptable salt, ester, stereoisomer, solvate, prodrug, or the aforementioned pharmaceutical composition, in the preparation of CDK kinase inhibitors.

A fifth objective of the present invention is to provide the use of a compound of formula (I), or a pharmaceutically acceptable salt, ester, stereoisomer, solvate, prodrug, or the aforementioned pharmaceutical composition, in the preparation of drugs for treating and/or preventing CDK-related diseases.

In a further embodiment, the CDK-related diseases are cancers.

Also provided herein is a method for inhibiting cell proliferation in vitro or in vivo, comprising contacting the cell with an effective amount of a compound of formula (I), as defined herein, or a pharmaceutically acceptable salt, solvate, or pharmaceutical composition thereof.

Also provided herein is a method for treating CDK-related diseases or disorders in a patient in need thereof, comprising administering to the patient a therapeutically effective amount of a compound of formula (I), as defined herein, or a pharmaceutically acceptable salt, solvate, or pharmaceutical composition thereof.

Also provided herein is a method for treating cancer and/or inhibiting cancer metastasis associated with specific cancers in a patient in need thereof, comprising administering to the patient a therapeutically effective amount of a compound of formula (I), as defined herein, or a pharmaceutically acceptable salt, solvate, or pharmaceutical composition thereof.

Also provided herein is a compound of formula (I), as defined herein, or a pharmaceutically acceptable salt, solvate, or pharmaceutical composition thereof, for use in therapy.

Also provided herein is a compound of formula (I), as defined herein, or a pharmaceutically acceptable salt, solvate, or pharmaceutical composition thereof, for use in the treatment of cancer and/or inhibition of cancer metastasis associated with specific cancers.

Also provided herein is a compound of formula (I), or a pharmaceutically acceptable salt or solvate thereof, for use in inhibiting CDK kinase activity.

Also provided herein is a compound of formula (I), as defined herein, or a pharmaceutically acceptable salt, solvate, or pharmaceutical composition thereof, for use in the treatment of CDK-related diseases or disorders.

Also provided herein is the use of a compound of formula (I), as defined herein, or a pharmaceutically acceptable salt or solvate thereof, in the manufacture of a medicament for the treatment of cancer and/or inhibition of cancer metastasis associated with specific cancers.

Also provided herein is the use of a compound of formula (I), as defined herein, or a pharmaceutically acceptable salt or solvate thereof, in the manufacture of a medicament for inhibiting CDK kinase activity.

Also provided herein is the use of a compound of formula (I), as defined herein, or a pharmaceutically acceptable salt or solvate thereof, in the manufacture of a medicament for the treatment of CDK-related diseases or disorders.

Also provided herein is a method for treating cancer in a patient in need thereof, comprising: (a) determining whether the cancer is associated with abnormal regulation of expression, activity, or level of CDK gene, CDK kinase, or any one thereof (e.g., CDK-related cancer); and (b) if it is determined that the cancer is associated with abnormal regulation of expression, activity, or level of CDK gene, CDK kinase, or any one thereof (e.g., CDK-related cancer), administering to the patient a therapeutically effective amount of a compound of formula (I), or a pharmaceutically acceptable salt, solvate, or pharmaceutical composition thereof.

Also provided herein is a pharmaceutical combination for treating cancer in a subject in need thereof (e.g., a CDK-related cancer, such as a CDK-related cancer comprising one or more mutations conferring resistance to CDK inhibitors), comprising: (a) a compound of formula (I) or a pharmaceutically acceptable salt or solvate thereof; (b) an additional therapeutic agent; and (c) optionally, at least one pharmaceutically acceptable carrier, wherein the compound of formula (I) or its pharmaceutically acceptable salt or solvate and the additional therapeutic agent are formulated as separate compositions or dosages for simultaneous, separate, or sequential administration to treat cancer, and wherein the amounts of the compound of formula (I) and the additional therapeutic agent are together effective in treating cancer. Also provided herein is a pharmaceutical composition comprising such a combination. The present disclosure further provides a use of such a combination for the manufacture of a medicament for treating cancer. Also provided herein is a commercial package or product comprising such a combination, presented as a formulation for simultaneous, separate, or sequential administration; and a method for treating cancer in a subject in need thereof.

The present disclosure further provides a method for reversing or preventing acquired resistance to an anti-cancer agent, comprising administering to a patient at risk of developing or having acquired resistance to the anti-cancer agent a therapeutically effective amount of a compound of formula (I) or a pharmaceutically acceptable salt or solvate thereof. In some embodiments, a dose of an anti-cancer agent is administered to the patient (e.g., substantially concurrently with administration of a dose of the compound of formula (I) or a pharmaceutically acceptable salt or solvate thereof).

The present disclosure further provides a method for delaying and/or preventing the development of cancer resistance to an anti-cancer agent in a subject, comprising administering to the subject a therapeutically effective amount of a compound of formula (I) or a pharmaceutically acceptable salt or solvate thereof before, during, or after administration of an effective amount of the anti-cancer agent.

The present disclosure further provides a method of treating a subject having cancer and being at increased risk of developing resistance to an anti-cancer agent, comprising administering to the subject (a) a therapeutically effective amount of a compound of formula (I) before, during, or after administration of (b) a therapeutically effective amount of the anti-cancer agent.

Also provided is a method for treating a subject having a CDK-related cancer and harboring one or more mutations conferring increased resistance to a first CDK inhibitor, comprising administering a compound of formula (I) or a pharmaceutically acceptable salt or solvate thereof before, during, or after administration of another anti-cancer agent.

Also provided is a method of treating a subject with a CDK-related cancer, comprising administering a compound of formula (I) or a pharmaceutically acceptable salt or solvate thereof before, during, or after administration of another anti-cancer agent (e.g., a first CDK kinase inhibitor).

The present invention also provides a method for preventing and/or treating a tumor, comprising administering to a subject in need thereof a therapeutically effective amount of a compound represented by formula (I), or a tautomer, stereoisomer, or isotopologue thereof, or a pharmaceutically acceptable salt, polymorph, or solvate of any of the foregoing.

In some embodiments, the tumor may be selected from breast cancer, ovarian cancer, small cell lung cancer, acute myeloid leukemia, acute lymphoblastic leukemia, bladder cancer, colon cancer, prostate cancer, epithelial sarcoma, and soft tissue sarcoma.

In some embodiments, the subject is human.

The compound of formula (I) and its pharmaceutically acceptable salts and solvates are also suitable for treating CDK-related cancers.

The present disclosure also provides a method of treating a patient diagnosed with or identified as having a CDK-related cancer (e.g., any exemplary CDK-related cancer disclosed herein), comprising administering to the patient a therapeutically effective amount of a compound of formula (I) as defined herein, or a pharmaceutically acceptable salt or solvate thereof, or a pharmaceutical composition thereof.

Unless otherwise defined, the terms of the present invention are defined as follows:
The term "halogen" refers to -F (also referred to as "fluoro" herein), -Cl, -Br, and -I.

The term "deuterated C1-C3 alkyl" refers to a saturated straight or branched monovalent hydrocarbon group having one to three carbon atoms, in which one to three carbon atoms are substituted with deuterium. Examples include, but are not limited to, deuterated methyl, deuterated ethyl, deuterated n-propyl, and deuterated isopropyl.

The terms "C1-C3 alkyl," "C1-C6 alkyl," "C2-C6 alkyl," and "C3-C6 alkyl" refer to saturated straight or branched monovalent hydrocarbon groups having one to three, one to six, two to six, or three to six carbon atoms, respectively. Examples include, but are not limited to, methyl, ethyl, n-propyl, isopropyl, n-butyl, isobutyl, see-butyl, tert-butyl, 2-methyl-2-propyl, pentyl, neopentyl, and hexyl.

The term "C1-C6 alkoxy" refers to a saturated straight or branched monovalent alkoxy group having one to six carbon atoms, where the point of attachment is on the oxygen atom. Examples include methoxy, ethoxy, propoxy, isopropoxy, butoxy, and tert-butoxy.

The terms "hydroxy-C1-C6 alkyl" and "hydroxy-C2-C6 alkyl" refer to saturated straight or branched monovalent alkyl groups having one to six or two to six carbon atoms, respectively, in which one carbon atom is substituted with a hydroxyl group.

The terms "deuterated C1-C6 alkyl," "halogenated C1-C6 alkyl," and "cyano-C1-C6 alkyl" refer to saturated straight or branched monovalent alkyl groups having one to six carbon atoms, wherein one carbon atom is substituted with deuterium, a halogen, or a cyano group, respectively.

The term "heterocycle" refers to a non-aromatic monocyclic or bicyclic ring system that, in addition to carbon atoms, contains 2-4 heteroatoms selected from the group consisting of P and N atoms.

The term "aryl" refers to an aromatic monocyclic or polycyclic group containing 6-19 carbon atoms. Aryl includes, but are not limited to, groups such as unsubstituted or substituted phenyl, and unsubstituted or substituted naphthyl.

The term "treating" or "treatment" as used throughout this document is conventional, for example, referring to the management or care of an individual for the purpose of combating, alleviating, reducing, relieving, or improving a condition of a disease or disorder such as cancer.

The terms "individual" or "patient" include organisms capable of having a cell proliferative disorder or capable of benefiting from the administration of the compound of the present invention, such as humans and non-human animals. Preferred humans include patients suffering from or prone to cell proliferative disorders or related conditions as described herein. The term "non-human animals" includes vertebrates, such as mammals, including non-human primates, sheep, cows, dogs, cats, and rodents (e.g., mice), as well as non-mammals, such as chickens, amphibians, reptiles, etc.

The term "cell proliferation" includes undesired or uncontrolled proliferation involving cells. The compound of the present invention may be used to prevent, suppress, block, reduce, decrease, or control cell proliferation and/or cell division, and/or to induce apoptosis. The method comprises administering to an individual (including mammals, including humans) in need thereof an effective amount of the compound of the present invention or a pharmaceutically acceptable salt, isomer, polymorph, metabolite, hydrate, or solvate thereof for the treatment or prevention of said disorder.

Compared with the prior art, the beneficial effects of the present invention are as follows:The CDK7 kinase inhibitor compound of the present invention exhibits enzymatic and cellular level bioactivity superior to that of publicly disclosed compounds targeting the same class, and has better bioavailability. The compound of the present invention provides more options for novel anti-tumor drugs and has promising pharmaceutical application prospects.

### DETAILED DESCRIPTION

The present invention is further illustrated by the following embodiments, which are provided for the purpose of explanation only and shall not be construed as limiting the present invention in any way.

The representative examples below are intended to assist in illustrating the present invention and are not intended to, nor should they be interpreted to, limit the scope of the present invention. In fact, except for those examples that appear and are described herein, the entire content of the present invention document, including examples based on the scientific literature and patents cited herein, as well as various modifications and numerous further variations derived therefrom, are clear and apparent to those skilled in the art. It should also be understood that the citation of these references serves to support the description of the contents herein. The following examples contain important supplementary information, illustrations, and guidance, which may be applicable to various modifications and analogous situations within the scope of the present invention.

### Example 1:

The synthetic route of the compound shown in Scheme 8 of the present invention is as follows:

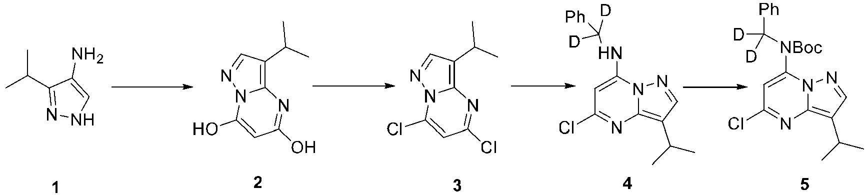

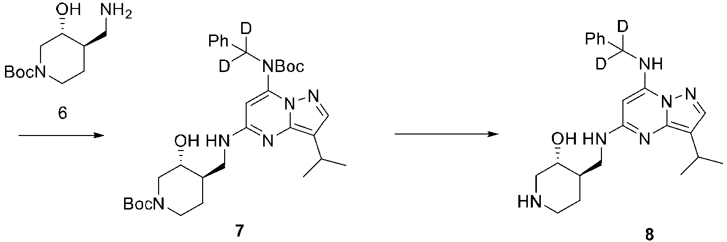

The preparation method of the above compound 2 is as follows:

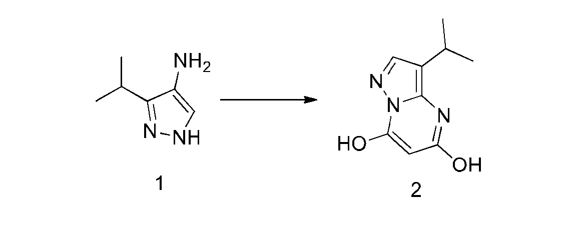

100 mL round-bottom flask was charged with compound 1 (2.0 g, 15.98 mmol), which was dissolved in ethanol at room temperature. Sodium ethoxide (3.26 g, 47.9 mmol) and diethyl malonate (3.33 g, 20.8 mmol) were then added. The mixture was heated to 80°C (internal temperature) and stirred for 15 hours. Upon completion of the reaction, the mixture was cooled to room temperature, and the pH was adjusted to 2.0 using 1 M HCl solution, resulting in the precipitation of a solid. The solid was filtered and washed with water to afford compound 2 as a white solid (2.8 g, 14.5 mmol), with a yield of 91%.

The preparation method of the above compound 3 is as follows:

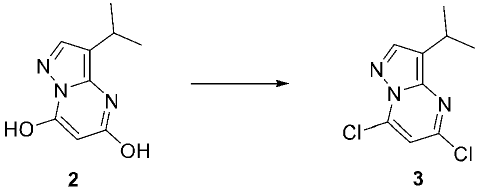

100 mL round-bottom flask was charged with phosphorus oxychloride (8 mL) and acetonitrile (7 mL) at room temperature. Then, compound 2 (500 mg, 2.6 mmol) was slowly added to the reaction solution. Subsequently, triethylamine (521.13 mg, 5.2 mmol) was added dropwise at 0°C. After the addition was complete, the reaction mixture was raised to 90°C and stirred overnight until the starting material was completely consumed. Upon completion of the reaction, the reaction mixture was concentrated, and then slowly quenched into ice water. The pH was adjusted to basic using solid sodium bicarbonate. The mixture was extracted with methyl tert-butyl ether, and the organic layer was concentrated to afford the crude product 3 as a reddish-brown solid.

The preparation method of the above compound 4 is as follows:

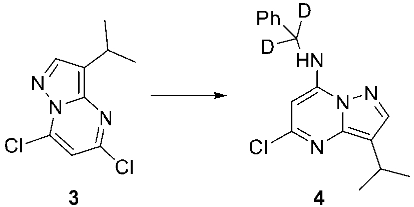

100 mL round-bottom flask was charged with compound 3 (500 mg, 2.17 mmol), deuterated benzylamine (521.02 mg, 4.78 mmol), and triethylamine (439.2 mg, 4.34 mmol), which were dissolved in ethanol and refluxed overnight until the starting material was completely consumed. Upon completion of the reaction, the mixture was evaporated to dryness, and compound **4** (491.5 mg, 1.64 mmol) was obtained by column chromatography (PE: EA = 10:1), with a yield of 78%.

The preparation method of the above compound **5** is as follows:

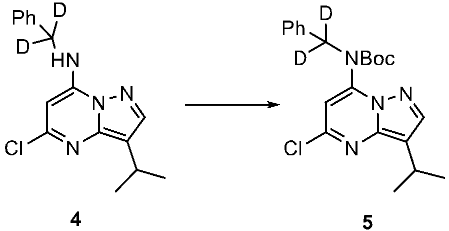

100 mL round-bottom flask was charged with compound 4 (491.5 mg, 1.64 mmol), di-tert-butyl dicarbonate (BoC₂O, 465.3 mg, 2.13 mmol), and THF. The mixture was stirred at room temperature for 2 hours until the starting material was completely consumed. Upon completion of the reaction, the mixture was evaporated to dryness, and compound **5** (616.2 mg, 1.54 mmol) was obtained by column chromatography (PE: EA = 10:1), with a yield of 94.3%.

The preparation method of the above compound **7** is as follows:

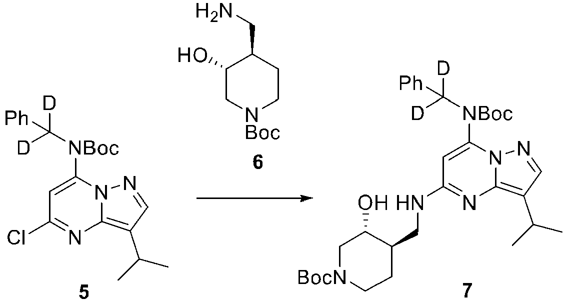

100 mL round-bottom flask was charged with compound 5 (500 mg, 1.25 mmol), Pd₂dba₃ (114.4 mg, 0.125 mmol), BINAP (233.5 mg, 0.38 mmol), and sodium tert-butoxide (180.2 mg, 1.88 mmol), which were dissolved in toluene (20 mL). The mixture was stirred at room temperature for 5 minutes, and then compound 6 (345 mg, 1.5 mmol) was slowly added. The mixture was then heated to 95°C and refluxed overnight. TLC was used to monitor the reaction until the starting material was consumed. Water and ethyl acetate were added to extract the reaction mixture, and the organic phase was collected. Compound 7 (370 mg, 0.62 mmol) was obtained by column chromatography (PE: EA = 1:1), with a yield of 50%. The preparation method of the above compound 8 is as follows:

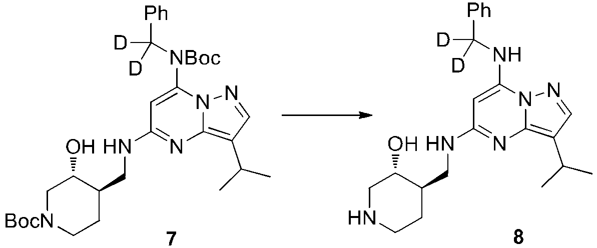

Compound **7** (370 mg, 0.62 mmol) was slowly added to 30 mL of hydrochloric acid in ethyl acetate in a 100 mL round-bottom flask, and stirred at room temperature for 2 hours until the starting material was completely consumed. The final product compound **8** (98 mg, 0.24 mmol) was obtained by thin-layer chromatography, with a yield of 40%.

¹H NMR (400 MHz, Chloroform-d) δ 7.65 (s, 1H), 7.39 - 7.30 (m, 5H), 6.46 (s, 1H), 4.98 (s, 1H), 4.80 - 4.71 (m, 1H), 4.30 (ddd, *J* = 14.8, 8.1, 2.9 Hz, 1H), 3.22 (ddd, *J =* 20.9, 10.5, 4.9 Hz, 2H), 3.13 - 2.98 (m, 2H), 2.92 (ddd, *J* = 14.6, 5.6, 1.8 Hz, 1H), 2.58 (td, *J =* 12.0, 2.8 Hz, 1H), 2.48 (t, *J =* 10.7 Hz, 1H), 1.60 - 1.41 (m, 3H), 1.29 (dd, *J =* 9.3, 6.9 Hz, 6H).

LC-MS [M+H⁺]397.37.

### Example 2

The synthetic route of the compound shown in Scheme **22** of the present invention is as follows:

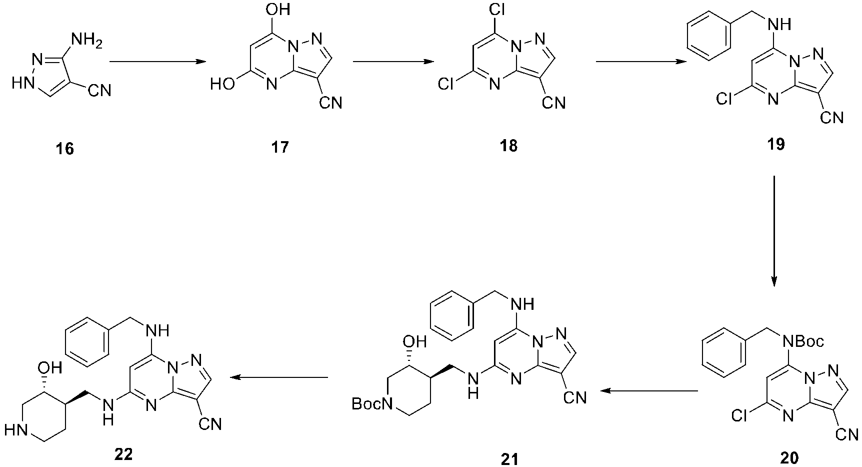

The preparation method of the above compound **17** is as follows:

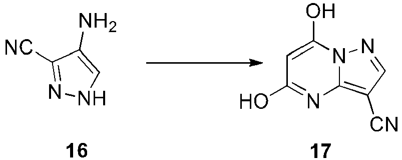

A 100 mL round-bottom flask was charged with compound **16** (2.0 g, 18.5 mmol), which was dissolved in ethanol at room temperature. Sodium ethoxide (3.78 g, 55.5 mmol) and diethyl malonate (3.85 g, 1.3 mmol) were added. The resulting mixture was heated to 80°C (internal temperature) and stirred for 15 h. After the completion of the reaction, the mixture was cooled to room temperature. The pH was adjusted to 2.0 using 1 M HCl solution, and a solid precipitated. The solid was filtered and washed with water to afford compound **17** as a white solid (1.7 g, 9.6 mmol), with a yield of 52%.

The preparation method of the above compound **18** is as follows:

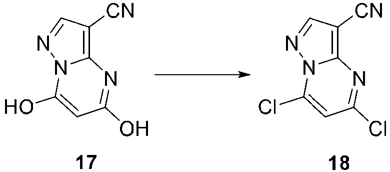

A 100 mL round-bottom flask was charged with phosphorus oxychloride (25 mL) and acetonitrile (24 mL) at room temperature. Compound **17** (1.7 g, 9.66 mmol) was slowly added to the reaction mixture. Then, triethylamine (1.96 g, 19.32 mmol) was added dropwise at 0°C. After the addition was completed, the reaction mixture was heated to 90°C and stirred overnight until the starting material was completely consumed. After the reaction was completed, the reaction mixture was concentrated. The mixture was slowly quenched into ice water. The pH was adjusted to basic using solid sodium bicarbonate. The mixture was extracted with methyl tert-butyl ether. The organic phase was concentrated to afford crude product **18** (1.2 g, 5.69 mmol) as a reddish-brown solid, with a yield of 59%.

The preparation method of the above compound **19** is as follows:

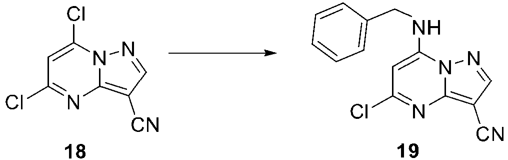

A 100 mL round-bottom flask was charged with compound **18** (1.2 g, 5.69 mmol), benzylamine (1.34 g, 12.5 mmol), and triethylamine (2.02 g, 11.38 mmol), which were dissolved in ethanol and refluxed overnight until the starting material was completely consumed. After the reaction was completed, the mixture was evaporated to dryness. Compound **19** (1.3 g, 4.5 mmol) was obtained by column chromatography (PE:EA = 10:1), with a yield of 79%.

The preparation method of the above compound **20** is as follows:

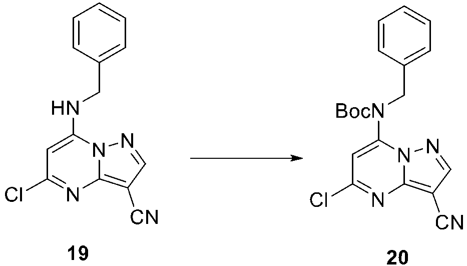

A 100 mL round-bottom flask was charged with compound **19** (1.3 g, 4.5 mmol), di-tert-butyl dicarbonate (BoC₂O, 5.1 g, 23.4 mmol), and DMAP (114.5 mg, 0.9 mmol), which were dissolved in THF and stirred at room temperature for 12 h until the starting material was completely consumed. After the reaction was completed, the mixture was evaporated to dryness. Compound **20** (1.6 g, 4.23 mmol) was obtained by column chromatography (PE: EA = 10:1), with a yield of 94%.

The preparation method of the above compound **21** is as follows:

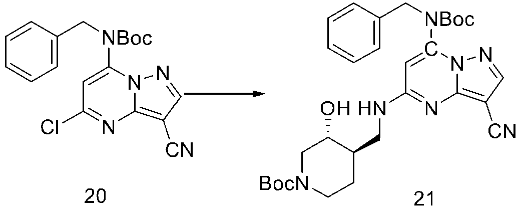

A 100 mL round-bottom flask was charged with compound **20** (400 mg, 1.04 mmol), Pd₂dba₃ (95.16 mg, 0.11 mmol), BINAP (194.28 mg, 0.312 mmol), and sodium tert-butoxide (151.36 mg, 1.58 mmol), which were dissolved in toluene (20 mL). The mixture was stirred at room temperature for 5 minutes. Compound **6** (288.3 mg, 1.25 mmol) was then slowly added to the reaction mixture. The mixture was heated to 95°C and refluxed overnight. TLC monitoring showed no remaining starting material. The mixture was extracted with water and ethyl acetate. The organic layers were combined and purified by column chromatography (PE: EA = 1:1) to obtain the final product compound **21** (277.8 mg, 0.58 mmol), with a yield of 56%.

The preparation method of the above compound **22** is as follows:

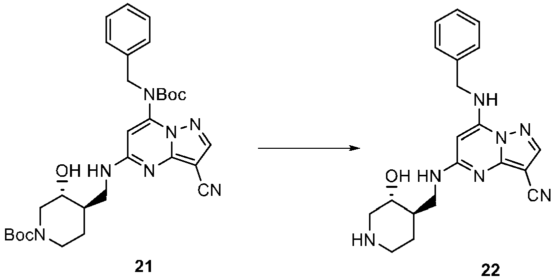

Compound **21** (277.8 mg, 0.58 mmol) was added in portions to a solution of hydrogen chloride in ethyl acetate in a 100 mL round-bottom flask. The reaction was stirred at room temperature for 2 h until the starting material was completely consumed. The reaction was neutralized with ammonia water and extracted with ethyl acetate. The organic phases were combined and purified by column chromatography to afford 45 mg of compound **22.**

¹H NMR (400 MHz, DMSO-d6) δ 9.11 (s, 1H), 8.28 (s, 1H), 7.47 (s, 1H), 7.39 - 7.32 (m, 4H), 7.27 (dd, *J* = 8.0, 5.0 Hz, 1H), 5.57 (s, 1H), 5.41 (s, 1H), 4.46 (s, 2H), 3.68 - 3.47 (m, 2H), 3.16 (td, *J =* 16.1, 14.1, 8.1 Hz, 3H), 2.65 (dt, *J* = 53.2, 11.6 Hz, 2H), 1.63 (d, *J =* 9.9 Hz, 2H), 1.52 - 1.15 (m, 3H).

LC-MS [M+H⁺]378.34.

### Example 3

The synthetic route of the compound shown in Scheme **33** of the present invention is as follows: 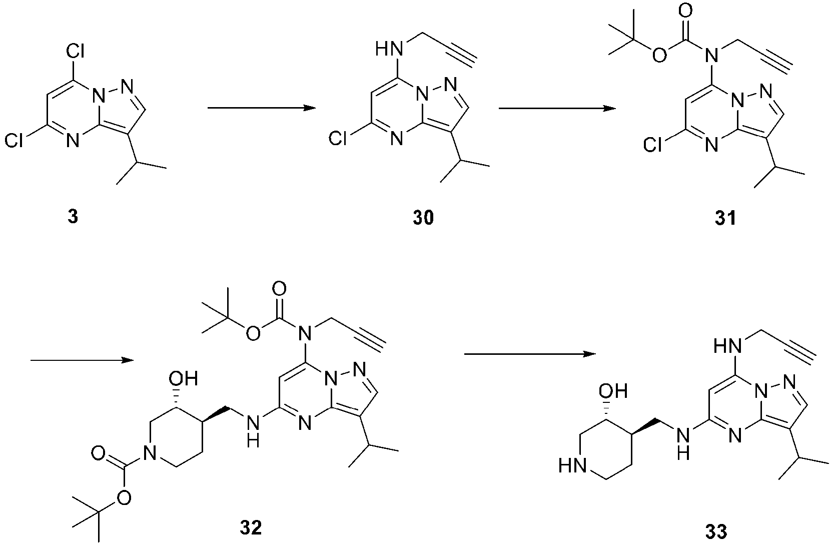 The preparation method of the above compound **30** is as follows:

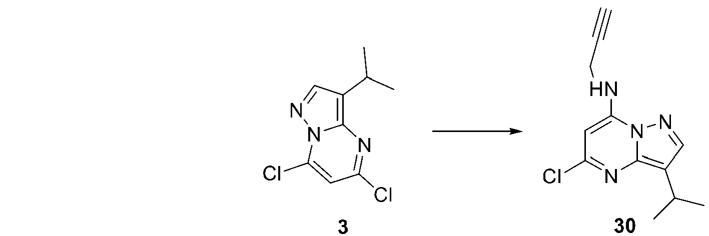

A 100 mL round-bottom flask was charged with compound 3 (500 mg, 2.17 mmol), propargylamine (119.5 mg, 2.17 mmol), and DIPEA (336.7 mg, 2.6 mmol), which were dissolved in isopropanol and stirred at room temperature overnight until the starting material was completely consumed. After the reaction was completed, the mixture was evaporated to dryness. Compound **30** (468.2 mg, 1.89 mmol) was obtained by column chromatography (PE: EA = 10:1), with a yield of 87%.

The preparation method of the above compound **31** is as follows:

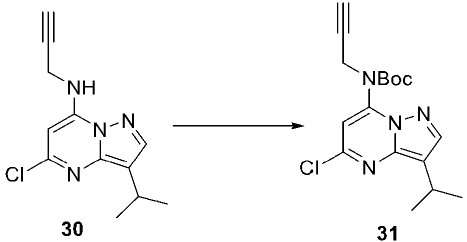

A 100 mL round-bottom flask was charged with compound **30** (468.2 mg, 1.89 mmol), di-tert-butyl dicarbonate (BoC₂O, 536.2 mg, 2.46 mmol), and DMAP (22 mg, 0.18 mmol), which were dissolved in THF and stirred at room temperature for 2 h until the starting material was completely consumed. After the reaction was completed, the mixture was evaporated to dryness. Compound **31** (618.3 mg, 1.78 mmol) was obtained by column chromatography (PE: EA = 10:1), with a yield of 94%.

The preparation method of the above compound **32** is as follows:

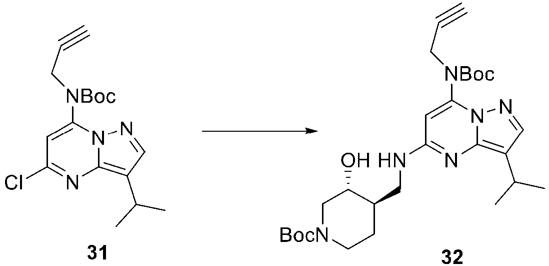

A 100 mL round-bottom flask was charged with compound **31** (618.3 mg, 1.78 mmol), compound **6** (409.4 mg, 1.78 mmol), and DIPEA (460.3 mg, 3.56 mmol), which were dissolved in DMAC. The mixture was heated to 130°C and refluxed overnight. TLC monitoring showed no remaining starting material. The mixture was extracted with water and ethyl acetate. The organic phases were combined and purified by column chromatography (PE: EA = 3:1) to afford the final product compound **32** (646.38 mg, 1.19 mmol), with a yield of 67%.

The preparation method of the above compound **33** is as follows:

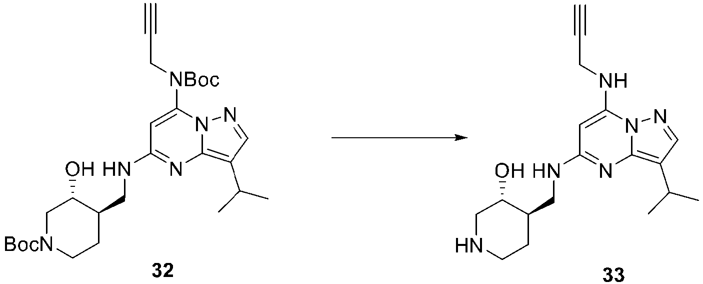

A solution of 3 mol/L methanesulfonic acid/dry dichloromethane solution in dry dichloromethane was prepared in a 100 mL round-bottom flask. Compound **32** (646.38 mg, 1.19 mmol) was added in portions to the reaction solution, and the mixture was stirred at room temperature for 2 h until the starting material was completely consumed. The reaction was neutralized with saturated potassium carbonate solution and extracted with dichloromethane. The organic phases were combined, and the product compound **33** (48 mg) was obtained by high-pressure purification.

¹H NMR (400 MHz, Chloroform-d) *δ* 7.72 - 7.57 (m, 1H), 6.34 (s, 1H), 5.52 (s, 1H), 5.27 (s, 1H), 4.25 (s, 1H), 3.99 (d, *J =* 30.0 Hz, 2H), 3.39 - 3.33 (m, 1H), 3.28 - 3.16 (m, 2H), 3.08 - 2.96 (m, 2H), 2.74 - 2.57 (m, 2H), 2.34 (dd, *J =* 22.7, 2.5 Hz, 1H), 1.55 (dd, *J =* 26.2, 11.9 Hz, 3H), 1.28 - 1.23 (m, 6H).

LC-MS [M+H⁺]343.30.

### Example 4

The synthetic route of the compound shown in Scheme **38** of the present invention is as follows:

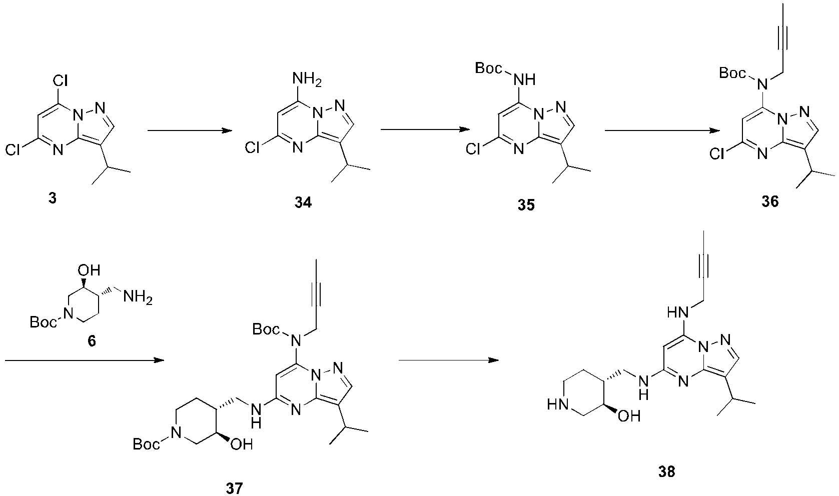

The preparation method of the above compound **34** is as follows:

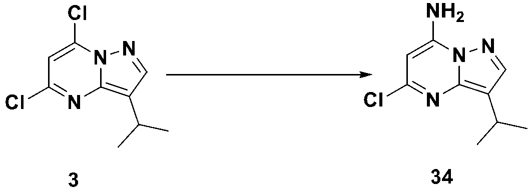

Accurately weigh compound **3** (1.05 g, 4.56 mmol) and aqueous ammonia (25%) (10.86 g, 77.5 mmol) and sequentially add them into a 75 mL sealed tube. Seal the tube and maintain the temperature at 80°C for 6 h.

LC-MS analysis confirmed the complete consumption of the starting material. The reaction mixture was cooled to room temperature, then stirred at 0°C for 15 min to precipitate more solids. The mixture was filtered under vacuum, and the filter cake was washed with water and dried under vacuum. The filter cake was dissolved in methyl tert-butyl ether, the aqueous phase was separated, and the organic phase was dried over anhydrous sodium sulfate and filtered. The filtrate was concentrated under vacuum to give off-white solid **34** (0.8 g, 3.80 mmol), with a yield of 83.3%.

The preparation method of the above compound **35** is as follows:

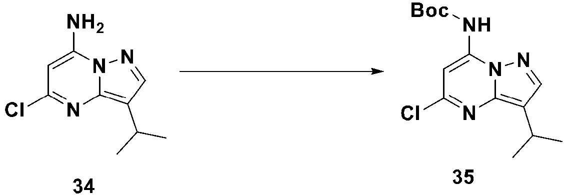

Accurately weigh compound **34** (610 mg, 2.9 mmol), THF (6.0 g), Boc2O (664 mg, 3.05 mmol), triethylamine (293 mg, 2.9 mmol), and 4-dimethylaminopyridine (DMAP) (35 mg, 0.29 mmol), and sequentially add them into a 100 mL single-neck flask. Stir the mixture at room temperature for 4 h.

TLC analysis confirmed the complete consumption of the starting material. The reaction solution was concentrated under vacuum. The residue was dissolved in a small amount of dichloromethane and purified by column chromatography (PE: EA = 20:1) to obtain white solid compound **35** (800 mg, 2.58 mmol), with a yield of 88.9%.

The preparation method of the above compound **36** is as follows:

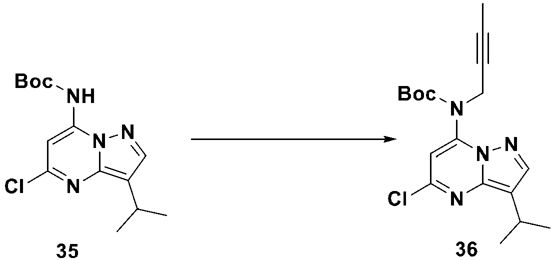

Accurately weigh compound **35** (400 mg, 1.29 mmol), ACN (4.0 g), 1-bromo-2-butyne (189 mg, 1.42 mmol), and cesium carbonate (463 mg, 1.42 mmol), and sequentially add them into a 15 mL sealed tube. Maintain the temperature at 80°C for 2 h.

TLC analysis confirmed the complete consumption of the starting material. The reaction mixture was cooled to room temperature and filtered. The filter cake was washed with DCM and dried under vacuum. The filtrate was concentrated under vacuum. The residue was dissolved in a small amount of dichloromethane and purified by column chromatography (PE: EA = 20:1) to obtain white solid compound **36** (450 mg, 1.24 mmol), with a yield of 96.3%.

The preparation method of the above compound **37** is as follows:

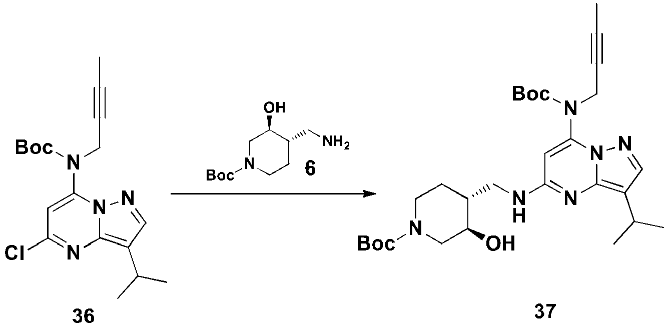

Accurately weigh compound **36** (400 mg, 1.1 mmol), dimethylacetamide (4.0 g), compound **6** (330 mg, 1.43 mmol), and DIPEA (427 mg, 3.3 mmol), and sequentially add them into a 15 mL sealed tube. The reaction was conducted under argon protection, sealed, and heated to 130°C for 20 h.

TLC analysis confirmed the complete consumption of the starting material. The reaction was cooled to room temperature and poured into water, extracted three times with EA. The organic phases were combined, washed with saturated brine, dried over anhydrous sodium sulfate, and filtered. The filtrate was concentrated under vacuum. The residue was dissolved in a small amount of DCM and purified by column chromatography with gradient elution (PE: EA = 5:1 → 3:1 → 2:1) to obtain light yellow liquid compound **37** (510 mg, 1.07 mmol), with a yield of 97.8%.

The preparation method of the above compound **38** is as follows:

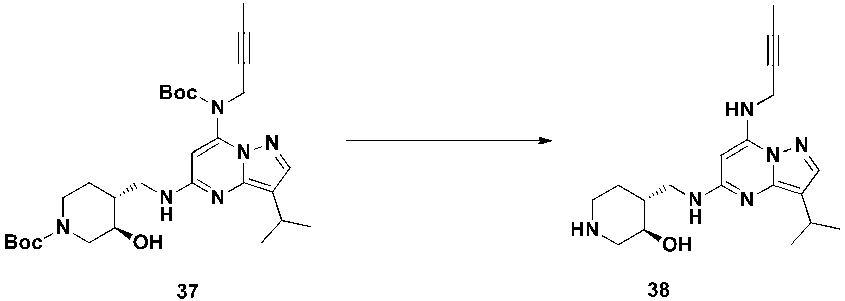

Accurately weigh compound **37** (510 mg, 0.917 mmol), dry DCM (5 mL), and methanesulfonic acid (528 mg, 5.5 mmol), and sequentially add them into a 25 mL single-neck flask. The reaction was conducted under argon protection at room temperature for 3 h.

LC-MS analysis confirmed the complete consumption of the starting material and complete formation of the intermediate (mono-deprotected Boc). The reaction mixture was poured into saturated sodium bicarbonate solution, and the aqueous phase was measured to have pH = 8-9. Extracted three times with EA. The organic phases were combined, washed with saturated brine, dried over anhydrous sodium sulfate, and filtered. The filtrate was concentrated under vacuum. A small amount of ether was added to the residue to precipitate the solid. The mixture was stirred at room temperature for 15 mins, filtered, and the filter cake was dried under vacuum to afford light yellow solid compound **38** (75 mg), with a yield of 23%.

¹H NMR (400 MHz, Chloroform-*d*) δ 7.67 (s, 1H), 6.20 (s, 1H), 5.17 (s, 1H), 4.91 (t, *J* = 6.9 Hz, 1H), 4.36 (ddd, *J =* 14.8, 7.9, 3.3 Hz, 1H), 4.01 (s,2H), 3.55 (td, *J =* 10.4, 3.8 Hz, 1H), 3.49 (t, *J* = 7.0 Hz, 1H), 3.41 (dd, *J =* 11.9, 4.5 Hz, 1H), 3.33 (d, *J =* 12.4 Hz, 1H), 3.16 - 2.98 (m, 2H), 2.74 (td, *J* = 12.4, 3.3 Hz, 1H), 2.65 (t, *J =* 11.4 Hz, 1H), 1.83 (t, *J =* 2.4 Hz, 3H), 1.79 - 1.65 (m, 2H), 1.59 (d, *J* = 11.3 Hz, 1H), 1.30 (dd, *J* = 11.4, 6.9 Hz, 6H).

### Example 5

The synthetic route of the compound shown in Scheme **41** of the present invention is as follows:

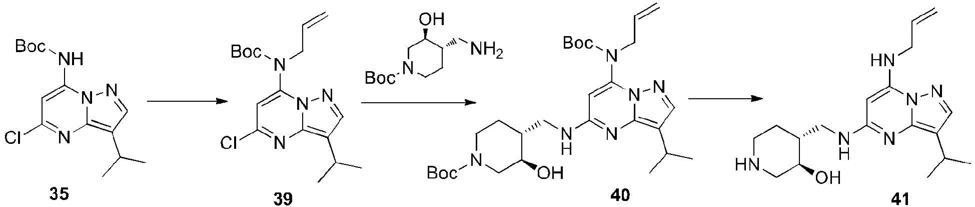

The preparation method of the above compound **39** is as follows:

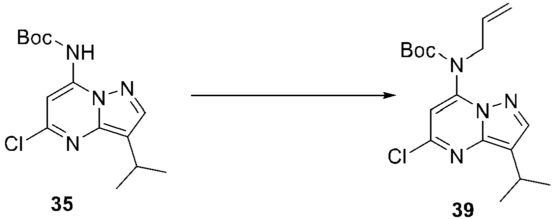

Accurately weigh compound **35** (300 mg, 0.97 mmol), ACN (4.0 g), allyl bromide (130 mg, 1.07 mmol), and cesium carbonate (350 mg, 1.07 mmol), and sequentially add them into a 15 mL sealed tube. Maintain the temperature at 80°C for 2 h.

TLC analysis confirmed the complete consumption of the starting material. The reaction mixture was cooled to room temperature and filtered. The filter cake was washed with DCM and dried under vacuum. The filtrate was concentrated under vacuum. The residue was dissolved in a small amount of DCM and purified by column chromatography (PE: EA = 20:1) to obtain white solid compound **39** (303 mg, 0.869 mmol), with a yield of 89.6%.

The preparation method of the above compound **40** is as follows:

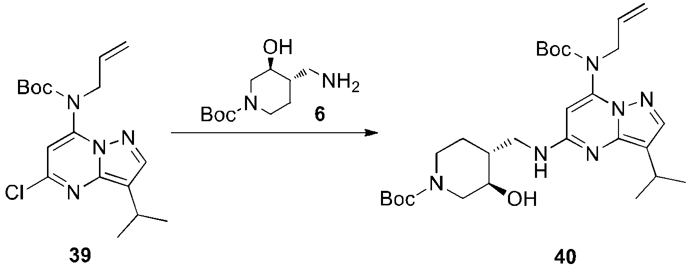

Accurately weigh compound **39** (300 mg, 0.86 mmol), DMAc (5.0 g), compound **6** (256 mg, 1.12 mmol), and DIPEA (333 mg, 2.58 mmol), and sequentially add them into a 15 mL sealed tube. The reaction was conducted under argon protection, sealed, and heated to 130°C for 7 h.

TLC analysis confirmed the complete consumption of the starting material. The reaction was cooled to room temperature and poured into water, extracted three times with EA. The organic phases were combined, washed with saturated brine, dried over anhydrous sodium sulfate, and filtered. The filtrate was concentrated under vacuum. The residue was dissolved in a small amount of DCM and purified by column chromatography with gradient elution (PE: EA = 4:1 → 2.5:1) to afford light yellow liquid compound **40** (350 mg), with a yield of 75.1%.

The preparation method of the above compound **41** is as follows:

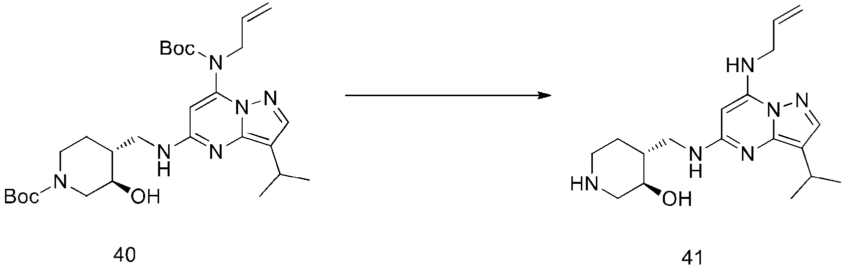

Accurately weigh compound **40** (300 mg, 0.643 mmol), dry DCM (4 mL), and methanesulfonic acid (370 mg, 3.86 mmol), and sequentially add them into a 25 mL single-neck flask. The reaction was conducted under argon protection at room temperature for 3 h.

LC-MS analysis confirmed the complete consumption of the starting material and complete formation of the intermediate (mono-deprotected Boc). The reaction mixture was poured into saturated sodium bicarbonate solution, and the aqueous phase was measured to have pH = 8-9. Extracted three times with EA. The organic phases were combined, washed with saturated brine, dried over anhydrous sodium sulfate, and filtered. The filtrate was concentrated under vacuum. A small amount of ether was added to the residue to precipitate the solid. The mixture was stirred at room temperature for 15 min, filtered, and the filter cake was dried under vacuum to afford light yellow solid compound **41** (68 mg), with a yield of 30%.

¹H NMR (400 MHz, Chloroform-d) δ 7.67 (s, 1H), 6.19 (d, *J =* 6.4 Hz, 1H), 5.91 (tt, *J* = 10.6, 5.3 Hz, 1H), 5.30 (dd, *J* = 30.8, 13.8 Hz, 2H), 5.02 (s, 1H), 4.71 (s, 1H), 4.38 (dd, *J =* 14.3, 8.4 Hz, 1H), 3.92 (s, 2H), 3.53 - 3.18 (m, 2H), 3.16 - 3.02 (m, 2H), 2.97 (dd, *J =* 14.7, 5.3 Hz, 1H), 2.57 (dt, *J =* 43.4, 11.3 Hz, 2H), 1.71 - 1.41 (m, 3H), 1.31 (d, *J* = 7.6 Hz, 6H).

### Example 6

The synthetic route of the compound shown in Formula **45** of the present invention is as follows:

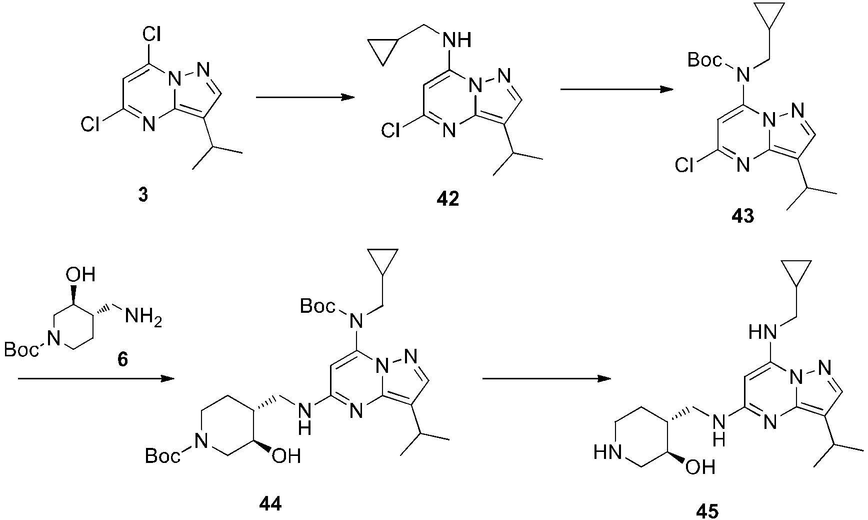

Preparation method of the above compound **42:**

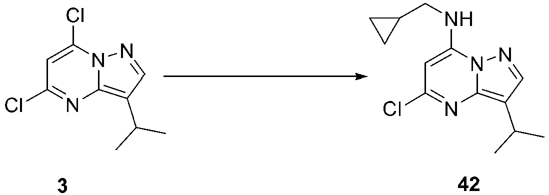

Accurately weigh compound **3** (500 mg, 2.17 mmol), ethanol (6 g), cyclopropylmethylamine (300 mg, 4.34 mmol), and triethylamine (438 mg, 4.34 mmol), and sequentially add them into a 15 mL sealed tube. Seal the tube, control the temperature at 80°C, and react for 3 h.

Detect by LC-MS: the starting material has completely reacted. Concentrate the reaction mixture under reduced pressure. Dissolve the residue in a small amount of DCM, purify by column chromatography (petroleum ether : ethyl acetate = 20:1), to afford off-white solid **42** (490 mg, 1.86 mmol), with a yield of 85.5%.

Preparation method of the above compound **43:**

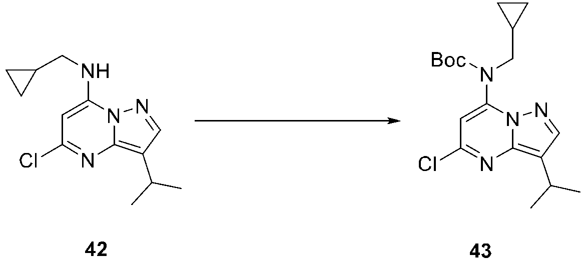

Accurately weigh compound **42** (490 mg, 1.85 mmol), THF (4.0 g), Boc₂O (484 mg, 2.22 mmol), TEA (280 mg, 2.78 mmol), and DMAP (23 mg, 0.19 mmol), and sequentially add them into a 100 mL single-necked flask. Stir at room temperature for 20 h.

Detect by TLC: the starting material has completely reacted. Concentrate the reaction mixture under reduced pressure. Dissolve the residue in a small amount of DCM, purify by column chromatography (PE: EA = 30:1), to afford pale yellow oily compound **43** (600 mg, 1.64 mmol), with a yield of 88.9%.

Preparation method of the above compound **44:**

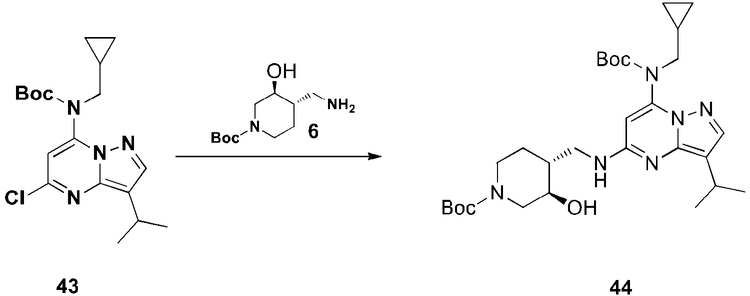

Accurately weigh compound **43** (600 mg, 1.66 mmol), DMAc (6.0 g), compound **6** (492 mg, 2.14 mmol), and DIPEA (638 mg, 4.93 mmol), and sequentially add them into a 75 mL sealed tube under argon protection. Seal the tube and control the temperature at 130°C, react for 4 h.

Detect by TLC: the starting material has completely reacted. Cool to room temperature, pour the reaction mixture into water, extract with ethyl acetate three times, combine organic layers, wash with saturated brine, dry over anhydrous sodium sulfate, filter, and concentrate under reduced pressure. Dissolve the residue in a small amount of DCM, purify by column chromatography with gradient elution (PE:EA = 4:1→2:1), to afford pale yellow liquid **44** (680 mg, 1.23 mmol), with a yield of 73.9%.

Preparation method of the above compound **45:**

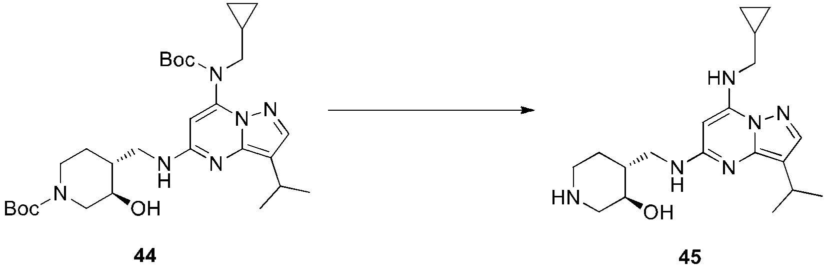

Accurately weigh compound **44** (100 mg, 0.18 mmol), ethyl acetate (1 mL), and HCl/ethyl acetate (3 M) (3 mL, 9 mmol), and sequentially add them into a 25 mL single-necked flask under argon protection. Stir at room temperature for 6 h.

Detect by LC-MS: the starting material has completely reacted and the intermediate (deprotection of one Boc group) has completely reacted. Pour the reaction mixture into saturated sodium bicarbonate solution and adjust the aqueous phase to pH 8-9. Extract with ethyl acetate three times, combine organic layers, wash with saturated brine, dry over anhydrous sodium sulfate, filter, and concentrate under reduced pressure. Dissolve the residue in a small amount of DCM, purify by column chromatography with gradient elution (DCM: MeOH = 10:1-5:1-4:1), to afford off-white solid **45** (33 mg, 0.09 mmol), with a yield of 51.6%.

¹H NMR (400 MHz, Chloroform-d) δ 7.67 (s, 1H), 6.10 (s, 1H), 5.02 (s, 1H), 4.72 (dd, *J* = 8.2, 5.5 Hz, 1H), 4.38 (ddd, *J =* 14.9, 8.2, 2.6 Hz, 1H), 3.31 (td, *J =* 11.0, 9.5, 4.4 Hz, 1H), 3.25 (dd, *J =* 11.4, 4.5 Hz, 1H), 3.15 - 3.05 (m, 4H), 2.97 (dd, *J =* 14.6, 5.4 Hz, 1H), 2.70 - 2.60 (m, 1H), 2.54 (t, *J =* 10.8 Hz, 1H), 1.68 - 1.59 (m, 1H), 1.57 - 1.51 (m, 2H), 1.39 (s, 1H), 1.35 - 1.23 (m, 6H), 0.70 - 0.56 (m, 2H), 0.32 (q, *J =* 5.2 Hz, 2H).

LC-MS : [M+H]⁺ = 359.43, [M+2H]²⁺ /2= 180.30.

### Biological Assays:

The following assay methods were used to evaluate the biological activity of the compounds prepared in the present invention in comparison with a known CDK inhibitor (CT7001, developed by Emory University and self-prepared), demonstrating that the compounds prepared in the present invention exhibit CDK7 kinase inhibitory activity and possess superior enzymatic and cellular level biological activity compared to publicly disclosed compounds targeting the same target.

### Experiment 1: Pharmacokinetic Study in ICR Mice

Male ICR mice (body weight 18-20 g) were administered the known CDK inhibitor CT7001, compound 33 of the present invention, and compound 41 of the present invention, respectively, by oral gavage and intravenous (tail vein) injection at a dose of 10 mg/kg. Each group consisted of 12 animals. The dosing vehicle was 10% DMSO + 10% polyoxyethylene castor oil + 80% physiological saline solution. The oral gavage group was fasted for approximately 12 hours prior to dosing and allowed food 4 hours after administration. Water was not restricted during the experiment. Approximately 0.2 mL of blood was collected from the orbital sinus at the following time points: pre-dose and 5, 15, 30 minutes, 1, 2, 4, 6, 8, and 24 hours post-dose. Blood was collected into EP tubes containing EDTA-K2 as anticoagulant and placed on ice. Plasma was separated by centrifugation at 3500 rpm for 5 minutes at 4°C within 1 hour of blood collection. Plasma samples were stored at -20°C until analysis. Plasma concentrations of CT7001, 33, and 41 were quantitatively analyzed using LC-MS/MS (liquid chromatography-tandem mass spectrometry). Pharmacokinetic parameters were calculated using WinNonlin software.

As shown in Table 1, compound 41 prepared in the present invention exhibited better oral bioavailability compared with CT7001 after oral administration.

**Table 1 Pharmacokinetic Parameters of Oral Administration Group**

| PK Parameter | Unit | CT7001 | **33** | **41** |
|---|---|---|---|---|
| | | po.10mg/kg | po.10mg/kg | po.10mg/kg |
| T_{1/2} | h | 2.5 | 2.9 | 1.5 |
| Tₘₐₓ | h | 2.0 | 0.5 | 2.0 |
| Cₘₐₓ | ng/mL | 99 | 26 | 150 |
| Tlast | h | 8 | 6 | 8 |
| Clast | ng/mL | 31 | 5 | 8 |
| MRTlast | H | 3.6 | 2.4 | 2.5 |
| AUClast | h*ng/mL | 541 | 65 | 445 |
| AUCINF_obs | h*ng/mL | 653 | 88 | 462 |
| Vz | mL/kg | 55150 | 482301 | 46177 |
| Cl | mL/h/kg | 255 | 1895 | 361 |
| Bioavailability (F) | % | 7.8 | 5.5 | 15 |

**Table 2. Pharmacokinetic Parameters of Intravenous Administration Group**

| PK Parameter | Unit | CT7001 | **33** | **41** |
|---|---|---|---|---|
| | | iv.10mg/kg | iv.10mg/kg | iv10mg/kg |
| T_{1/2} | H | 1.5 | 10.0 | 2.3 |
| Cₘₐₓ | ng/mL | 17402 | 990 | 4365 |
| Tlast | H | 8 | 24 | 8 |
| Clast | ng/mL | 126 | 6 | 19 |
| MRTlast | H | 1.3 | 2.4 | 1.1 |
| AUClast | h*ng/mL | 8123 | 1510 | 2961 |
| AUCINF_obs | h*ng/mL | 8401 | 1602 | 3025 |
| Vz | mL/kg | 2621 | 89985 | 10945 |
| Cl | mL/h/kg | 20 | 104 | 55 |
| Vss | mL/kg | 1910 | 27827 | 4219 |

As shown in Table 2, compound 33 prepared in the present invention exhibited a longer half-life after intravenous administration compared with CT7001, which may allow for reduced dosing frequency. Additionally, compounds 33 and 41 showed higher steady-state apparent volume of distribution (Vss) in mice following intravenous administration, indicating a wider tissue distribution of the drug in vivo.

### Experiment 2: Assay for Inhibition of CDK7/CycH/MAT1 Kinase Activity by the Prepared Compounds

The CDK7/CycH/MAT1 protein was thawed on ice, and the remaining reagents were equilibrated to room temperature. The CDK7/CycH/MAT1 protein and ATP/substrate were diluted to the corresponding concentrations using enzyme reaction buffer (50 mM Hepes, 10 mM MgCl₂, 0.01% Brij35, 1 mM EGTA, 2 mM DTT). The test compounds were added to a 384-well white low-volume enzyme assay plate at 20 nL per well, followed by the addition of the prepared CDK7/CycH/MAT1 protein (2 µL/well), mixed well, and incubated at 25°C for 10 minutes. The reaction was initiated by adding ATP/substrate and allowed to proceed at 25°C for 60 minutes. The final concentrations of each component in the enzyme reaction system were as follows: CDK7/CycH/MAT1 protein, 3.5 ng/µL; ATP, 30 µM; substrate, 0.1 µM. The concentrations of the test compounds ranged from high to low as follows: 5000, 1667, 556, 185, 62, 21, 7, 2, 1, and 0.3 nM. After completion of the enzymatic reaction, 4 µL of ADP-Glo Reagent was added to each well and incubated at 25°C for 40 minutes. Subsequently, 8 µL of Kinase Detection Reagent was added to each well and incubated at25°C for another 40 minutes. Chemiluminescence signals were recorded using a microplate reader, and the data were used to generate dose-response curves and calculate IC₅₀ values.

As shown in Table 3, compounds 8, 22, 33, and 41 all exhibited IC₅₀ values of less than 10 nM, indicating that the compounds prepared in the present invention possess inhibitory activity against CDK7/CycH/MAT1 kinase.

**Table 3. Enzymatic Activity Inhibition Data**

| Compound | IC50 (nM) |
|---|---|
| CT-7001 | 7.69 |
| **8** | 7.26 |
| **22** | 4.31 |
| **33** | 2.70 |
| **38** | 18.94 |
| **41** | 6.30 |
| **45** | 21.22 |

### Experiment 3: Evaluation of Anti-Proliferative Activity of the Compounds on Tumor Cells

Tumor cell lines including HCT-116, MDA-MB-468, Jurkat, HCC70, and MDA-MB-436 were cultured in their respective growth media. Cells were seeded into 384-well plates at densities of 600, 300, 800, 800, and 1000 cells/well (in 18 µL/well), respectively, and incubated overnight at 37°C under 5% CO₂ (MDA-MB-468 cells were incubated overnight at 37°C under 100% air). Test sample stock solutions were prepared in DMSO at 10 mM and serially diluted 1:3 across 10 concentrations in 1% DMSO to prepare 10X intermediate dilution plates. Then, 2 µL/well of the 1oX intermediate dilution was added to the cell plates to achieve the final assay concentrations: 10000, 3333, 1111, 370, 123, 41, 14, 4.6, and 1.5 nM. HCT-116, Jurkat, and HCC70 cell plates were incubated for 3, 7, and 7 days at 37°C with 5% CO₂, respectively (MDA-MB-468 cell plates were incubated for 7 days at 37°C with 100% air). After treatment, Cell Titer-Glo reagent was removed from -20°C storage and equilibrated to room temperature. Then, 20 µL/well of Cell Titer-Glo reagent was added to each well and incubated for 10 minutes. After the 10-minute incubation, luminescence was measured using a microplate reader. Data were analyzed using Excel and the IC₅₀ values were calculated using GraphPad Prism 7.0 software.

As shown in Table 4, compound **41** demonstrated stronger anti-proliferative activity than CT7001 across all tested tumor cell lines.

**Table 4. Inhibition of Tumor Cell Proliferation by Compounds**

| Cell Line | IC₃₀ (nM) | | | |
|---|---|---|---|---|
| | HCC70 | HCT-116 | MDA-MB-468 | Jurkat |
| CT7001 | 518.9 | 463.7 | 602.2 | 473.3 |
| **8** | / | 231.7 | 350.4 | 329.4 |
| **22** | / | 3453.0 | 2910.0 | 4087.0 |
| **33** | 501.9 | 788.3 | 1101 | 200.8 |
| **38** | 968.8 | 391.3 | 765.9 | 858.8 |
| **41** | 357.6 | 117.3 | 313.8 | 340.8 |
| **45** | 596.4 | 429.4 | 851.8 | 1126 |

The foregoing is merely a preferred embodiment of the present invention. It should be noted that for those skilled in the art, various modifications and alterations may be made without departing from the principles of the present invention, and such modifications and alterations shall also be deemed to fall within the scope of protection of the present invention.

## Claims

1. A compound of formula (I) or a pharmaceutically acceptable salt, ester, stereoisomer, solvate, or prodrug thereof, 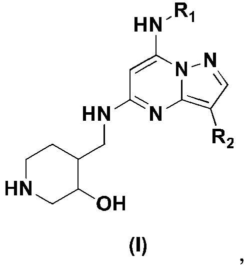
wherein R₁ is a substituted or unsubstituted deuterated C1-C3 alkyl, C1-C3 alkyl, C2-C6 alkenyl, C2-C6 alkynyl, or a substituted or unsubstituted C6-C10 aryl-C1-C6 alkyl;
the substituents are independently selected from H, D, halogen, hydroxyl, -CN, carbonyl, C1-C3 alkyl, C2-C3 alkenyl, C2-C3 alkynyl, deuterated C1-C3 alkyl, C1-C3 alkoxy, hydroxy-C1-C3 alkyl, fluoro-C1-C3 alkyl, cyano-C1-C3 alkyl, C3-C8 cycloalkyl, C3-C10 heterocyclyl, and C6-C10 aryl;
R2 is selected from halogen, hydroxyl, -CN, carbonyl, C1-C3 alkyl, C2-C3 alkenyl, C2-C3 alkynyl, deuterated C1-C3 alkyl, C1-C3 alkoxy, hydroxy-C1-C3 alkyl, fluoro-C1-C3 alkyl, and cyano-C1-C3 alkyl;
when R₁ is benzyl, R₂ is -CN.

2. The compound according to claim 1, or a pharmaceutically acceptable salt, ester, stereoisomer, solvate, or prodrug thereof, wherein R₁ is a substituted or unsubstituted C1-C3 alkyl, C2-C6 alkenyl, or C2-C6 alkynyl, or a substituted or unsubstituted C6-C10 aryl-C1-C6 alkyl.

3. The compound according to claim 1, or a pharmaceutically acceptable salt, ester, stereoisomer, solvate, or prodrug thereof, **characterized in that** R₂ is selected from -CN and C1-C3 alkyl.

4. The compound according to claim 1, or a pharmaceutically acceptable salt, ester, stereoisomer, solvate, or prodrug thereof, wherein R1 is a substituted or unsubstituted

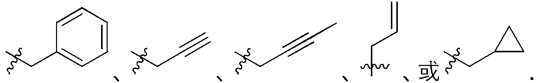

5. The compound according to claim 1, or a pharmaceutically acceptable salt, ester, stereoisomer, solvate, or prodrug thereof, wherein the compound is selected from:

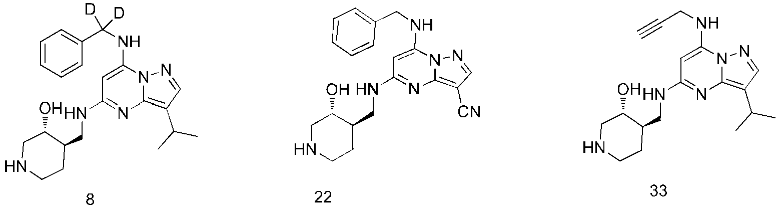

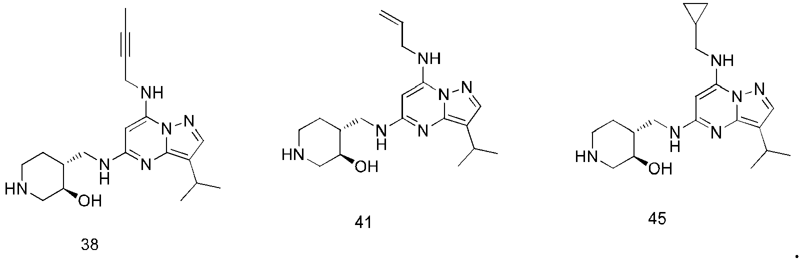

6. A pharmaceutical composition, **characterized in that** the pharmaceutical composition comprises the compound according to claim 1 or a pharmaceutically acceptable salt, ester, stereoisomer, solvate, or prodrug thereof.

7. The pharmaceutical composition according to claim 6, **characterized in that** the pharmaceutical composition further comprises another therapeutic agent and/or a pharmaceutically acceptable carrier.

8. Use of the compound according to claim 1 or a pharmaceutically acceptable salt, ester, stereoisomer, solvate, or prodrug thereof, or the pharmaceutical composition according to claim 6, in the preparation of a CDK kinase inhibitor.

9. Use of the compound according to claim 1 or a pharmaceutically acceptable salt, ester, stereoisomer, solvate, or prodrug thereof, or the pharmaceutical composition according to claim 6, in the preparation of a medicament for the treatment and/or prevention of CDK-related diseases.

10. The use according to claim 9, **characterized in that** the CDK-related disease is cancer.
